# EUROPEAN PATENT SPECIFICATION

(11) **EP 3 417 789 B1**
(45) Date of publication and mention of the grant of the patent: **16.09.2026**
(21) Application number: 16890726.9
(22) Date of filing: 04.05.2016
(51) Int. Cl.: A61B 8/00, G10K 11/34, G01S 7/52, G01S 15/89, A61B 8/14, B06B 1/02

(54) **METHOD FOR PERFORMING BEAMFORMING AND BEAMFORMER**
VERFAHREN ZUR DURCHFÜHRUNG VON STRAHLFORMUNG UND STRAHLFORMER
PROCÉDÉ DE MISE EN UVRE D'UNE FORMATION DE FAISCEAUX ET FORMEUR DE FAISCEAUX

(30) Priority: 16.02.2016 KR 20160017773
(43) Date of publication of application: 26.12.2018
(73) Proprietor: Samsung Medison Co., Ltd., Hongcheon-gun, Gangwon-do 25108 (KR)
(72) Inventor: HAN, Ho-san, Seoul 06966 (KR); KIM, Kang-sik, Seongnam-si Gyeonggi-do 13517 (KR)
(74) Representative: Arnold & Siedsma
(86) International application number: PCT/KR2016/004752
(87) International publication number: WO 2017/142134

(56) References cited:
- EP-A1- 2 919 271
- WO-A1-2017/114872
- JP-A- H06 319 732
- KR-A- 20110 022 440
- KR-A- 20130 068 529
- KR-A- 20150 057 175
- US-A1- 2010 049 042
- US-B1- 6 468 216

## Description

### BACKGROUND

### 1. Field

The present disclosure relates to methods and beamformers for performing beamforming.

### 2. Description of the Related Art

Ultrasound imaging apparatuses transmit ultrasound signals generated by transducers of a probe to an object and receive echo signals reflected from the object, thereby obtaining at least one image of an object or an internal part of the object (e.g., soft tissues or blood flow). In particular, ultrasound imaging apparatuses are used for medical purposes including observation of the interior of an object, detection of foreign substances, and diagnosis of damage to the object. Such ultrasound imaging apparatuses provide high stability, display images in real time, and are safe due to the lack of radioactive exposure, compared to X-ray apparatuses. Therefore, ultrasound imaging apparatuses are widely used together with other image diagnosis apparatuses including a computed tomography (CT) apparatus, a magnetic resonance imaging (MRI) apparatus, and the like.

Nakayama Yuta et al. ("Phasing adder, ultrasound probe, and acoustic sensor", EP2919271) discloses a phasing adder and an ultrasound probe therewith. The phasing adder includes a delay charge transferring unit, preferably a CCD, and a delay adding unit. The delay charge transferring unit obtains signal charge in an amount obtained without amplifying according to charge generated in each of a plurality of piezoelectric elements and holds the signal charge for a predetermined amount of time. The delay adding unit executes phasing adding of an amount of the signal charge held for the predetermined amount of time in the delay charge transferring unit.

Jeffry E. Powers et al. ("Ultrasonic diagnostic imaging of the coronary arteries", US6468216) discloses an ultrasonic imaging method and apparatus for imaging the coronary arteries of the heart. The vascular system is infused with an ultrasonic contrast agent.

### SUMMARY

Provided are methods and beamformers for performing beamforming.

Additional aspects will be set forth in part in the description which follows and, in part, will be apparent from the description, or may be learned by practice of the presented embodiments.

According to an aspect of an embodiment, there is provided a probe according to an embodiment of claim 1. The probe includes: a memory configured to store electric charges corresponding to echo signals reflected by an object and output the stored electric charges; a charger configured to generate an amount of electric charges corresponding to an amount of the output electric charges; and a controller configured to control at least one of a first velocity at which the electric charges are stored in the memory and a second velocity at which the electric charges are output from the memory.

According to an aspect of another embodiment, there is provided a method according to an embodiment of claim 5. The method of performing beamforming includes: receiving electric charges corresponding to echo signals reflected by an object and storing the received electric charges; outputting the stored electric charges at a preset velocity; and generating an amount of electric charges corresponding to an amount of the output electric charges and storing the generated electric charges.

### BRIEF DESCRIPTION OF THE DRAWINGS

These and/or other aspects will become apparent and more readily appreciated from the following description of the embodiments, taken in conjunction with the accompanying drawings in which:
FIGS. 1A and 1B illustrate examples of ultrasound diagnosis systems according to embodiments;
FIG. 2 is a block diagram of a configuration of an ultrasound diagnosis system according to an embodiment;
FIG. 3 is a block diagram of a configuration of a wireless probe according to an embodiment;
FIGS. 4A and 4B illustrate examples of one-dimensional (1D) and two-dimensional (2D) probes according to embodiments;
FIG. 5 illustrates a configuration of a probe according to an embodiment;
FIG. 6 is a diagram for explaining an example of groups and channels in each group according to an embodiment;
FIG. 7 illustrates a configuration of a beamformer according to an embodiment;
FIG. 8 illustrates an example of a configuration in which a beamformer is connected to a channel, according to an embodiment;
FIG. 9 illustrates a configuration of a sub-module according to an embodiment;
FIGS. 10A and 10B are diagrams for explaining an example in which a controller controls a velocity at which electric charges are input/output to/from a memory according to the number of scanlines; and
FIG. 11 is a flowchart of a method of performing beamforming according to an embodiment.

### DETAILED DESCRIPTION

The terms used in this specification are those general terms currently widely used in the art in consideration of functions regarding the inventive concept, but the terms may vary according to the intention of those of ordinary skill in the art, precedents, or new technology in the art. Also, some terms may be arbitrarily selected by the applicant, and in this case, the meaning of the selected terms will be described in detail in the detailed description of the present specification. Thus, the terms used in the specification should be understood not as simple names but based on the meaning of the terms and the overall description of the invention.

Throughout the specification, it will also be understood that when a component "includes" an element, unless there is another opposite description thereto, it should be understood that the component does not exclude another element and may further include another element. In addition, terms such as "... unit", "... module", or the like refer to units that perform at least one function or operation, and the units may be implemented as hardware or software or as a combination of hardware and software.

Throughout the specification, an "ultrasound image" refers to an image of an object, which is obtained using ultrasound waves, or an image showing a region of interest (ROI) included in the object. An ROI refers to a region of an object that a user desires to observe with more focused attention, and, for example, may be a region including a lesion. Furthermore, an "object" may be a human, an animal, or a part of a human or animal. For example, the object may be an organ (e.g., the liver, the heart, the womb, the brain, a breast, or the abdomen), a blood vessel, or a combination thereof. Also, the object may be a phantom. The phantom means a material having a density, an effective atomic number, and a volume that are approximately the same as those of an organism. For example, the phantom may be a spherical phantom having properties similar to a human body.

Throughout the specification, a "user" may be, but is not limited to, a medical expert, for example, a medical doctor, a nurse, a medical laboratory technologist, or a medical imaging expert, or a technician who repairs medical apparatuses.

Hereinafter, embodiments will be described in detail with reference to the accompanying drawings.

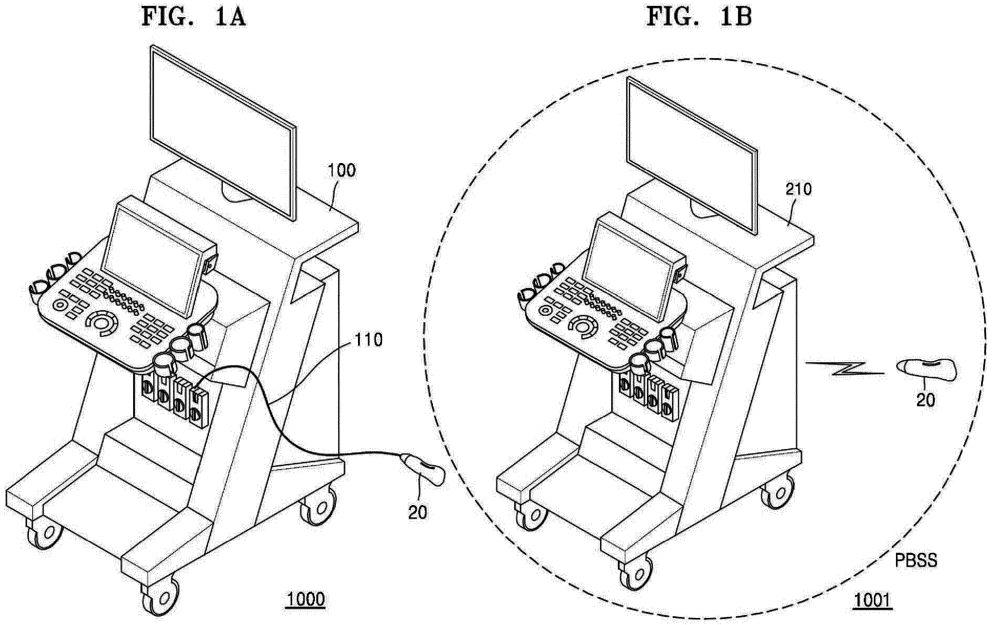

FIGS. 1A and 1B are diagrams showing examples of ultrasound diagnosis systems 1000 and 1001 according to embodiments.

Referring to FIG. 1A, in the ultrasound diagnosis system 1000, a probe 20 may be connected by wire to an ultrasound imaging apparatus 100. In other words, the probe 20 for transmitting and receiving ultrasound waves may be connected to a main body of the ultrasound diagnosis system 1000, i. e., the ultrasound imaging apparatus 100 via a cable 110.

Referring to FIG. 1B, in the ultrasound diagnosis system 1001, a probe 20 may be connected wirelessly to an ultrasound imaging apparatus 210. In other words, the probe 20 may be connected to the ultrasound imaging apparatus 210 via the same wireless network.

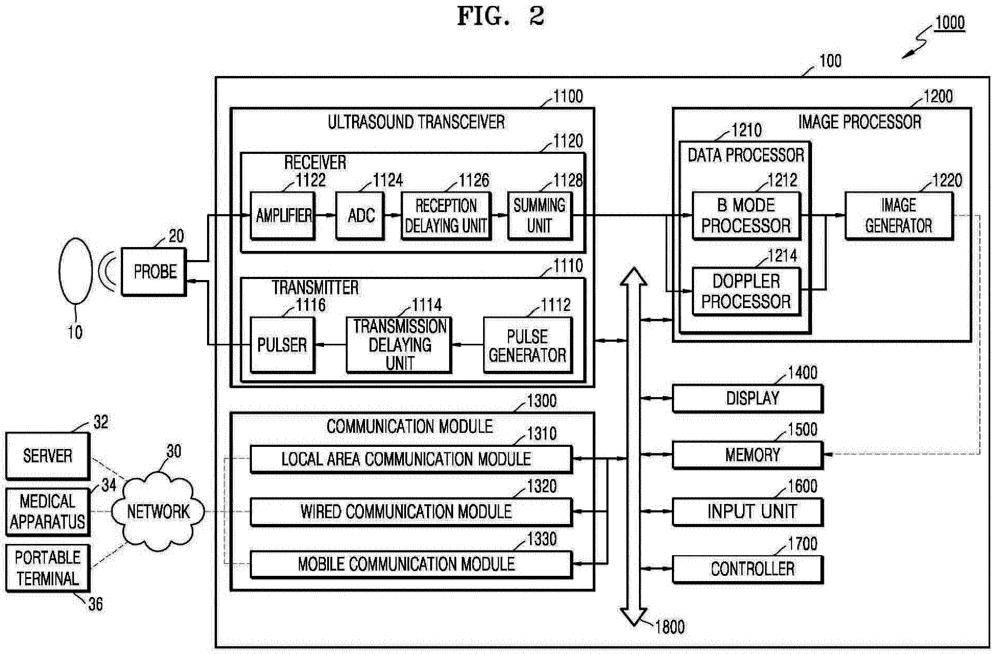

FIG. 2 is a block diagram of a configuration of an ultrasound diagnosis system 1002 according to an embodiment.

Referring to FIG. 2, the ultrasound diagnosis system 1002 may include a probe 20 and an ultrasound imaging apparatus 100. Referring to FIG. 1, the ultrasound imaging apparatus 100 may include a probe 20, an ultrasound transceiver 1100, an image processor 1200, a communication module 1300, a display 1400, a memory 1500, an input unit 1600, and a controller 1700, which may be connected to one another via buses 1800.

The ultrasound diagnosis system 1002 may be a cart type apparatus or a portable type apparatus. Examples of portable ultrasound imaging apparatuses may include, but are not limited to, a picture archiving and communication system (PACS) viewer, a smartphone, a laptop computer, a personal digital assistant (PDA), and a tablet PC.

The probe 20 transmits ultrasound signals to an object 10 (or to an ROI in the object 10) in response to a driving signal applied by the ultrasound transceiver 1100 and receives echo signals reflected by the object 10 (or by the ROI in the object 10). The probe 20 includes a plurality of transducers, and the plurality of transducers oscillate in response to electric signals and generate acoustic energy, that is, ultrasound waves. Furthermore, the probe 20 may be connected to the main body of the ultrasound diagnosis system 1002 by wire or wirelessly, and according to embodiments, the ultrasound diagnosis system 1002 may include a plurality of probes 20.

A transmitter 1110 supplies a driving signal to the probe 20. The transmitter 110 includes a pulse generator 1112, a transmission delaying unit 1114, and a pulser 1116. The pulse generator 1112 generates pulses for forming transmission ultrasound waves based on a predetermined pulse repetition frequency (PRF), and the transmission delaying unit 1114 delays the pulses by delay times necessary for determining transmission directionality. The pulses which have been delayed correspond to a plurality of piezoelectric vibrators included in the probe 20, respectively. The pulser 1116 applies a driving signal (or a driving pulse) to the probe 20 based on timing corresponding to each of the pulses which have been delayed.

A receiver 1120 generates ultrasound data by processing echo signals received from the probe 20. The receiver 120 may include an amplifier 1122, an analog-to-digital converter (ADC) 1124, a reception delaying unit 1126, and a summing unit 1128. The amplifier 1122 amplifies echo signals in each channel, and the ADC 1124 performs analog-to-digital conversion with respect to the amplified echo signals. The reception delaying unit 1126 delays digital echo signals output by the ADC 124 by delay times necessary for determining reception directionality, and the summing unit 1128 generates ultrasound data by summing the echo signals processed by the reception delaying unit 1166. In some embodiments, the receiver 1120 may not include the amplifier 1122. In other words, if the sensitivity of the probe 20 or the capability of the ADC 1124 to process bits is enhanced, the amplifier 1122 may be omitted.

The image processor 1200 generates an ultrasound image by scan-converting ultrasound data generated by the ultrasound transceiver 1100. The ultrasound image may be not only a grayscale ultrasound image obtained by scanning an object in an amplitude (A) mode, a brightness (B) mode, and a motion (M) mode, but also a Doppler image showing a movement of an object via a Doppler effect. The Doppler image may be a blood flow Doppler image showing flow of blood (also referred to as a color Doppler image), a tissue Doppler image showing a movement of tissue, or a spectral Doppler image showing a moving speed of an object as a waveform.

A B mode processor 1212 extracts B mode components from ultrasound data and processes the B mode components. An image generator 1220 may generate an ultrasound image indicating signal intensities as brightness based on the extracted B mode components 1212.

Similarly, a Doppler processor 1214 may extract Doppler components from ultrasound data, and the image generator 1220 may generate a Doppler image indicating a movement of an object as colors or waveforms based on the extracted Doppler components.

According to an embodiment, the image generator 1220 may generate a three-dimensional (3D) ultrasound image via volume-rendering with respect to volume data and may also generate an elasticity image by imaging deformation of the object 10 due to pressure. Furthermore, the image generator 1220 may display various pieces of additional information in an ultrasound image by using text and graphics. In addition, the generated ultrasound image may be stored in the memory 1500.

A display 1400 displays the generated ultrasound image. The display 1400 may display not only an ultrasound image, but also various pieces of information processed by the ultrasound imaging apparatus 1002 on a screen image via a graphical user interface (GUI). In addition, the ultrasound imaging apparatus 1000 may include two or more displays 1400 according to embodiments.

The communication module 1300 is connected to a network 30 by wire or wirelessly to communicate with an external device or a server. Furthermore, when the probe 20 is connected to the ultrasound imaging apparatus 1002 via a wireless network, the communication module 1300 may communicate with the probe 20.

The communication module 1300 may exchange data with a hospital server or another medical apparatus in a hospital, which is connected thereto via a PACS. Furthermore, the communication module 1300 may perform data communication according to the digital imaging and communications in medicine (DICOM) standard.

The communication module 1300 may transmit or receive data related to diagnosis of an object, e.g., an ultrasound image, ultrasound data, and Doppler data of the object, via the network 30 and may also transmit or receive medical images captured by another medical apparatus, e.g., a computed tomography (CT) apparatus, a magnetic resonance imaging (MRI) apparatus, or an X-ray apparatus. Furthermore, the communication module 1300 may receive information about a diagnosis history or medical treatment schedule of a patient from a server and utilizes the received information to diagnose the patient. Furthermore, the communication module 1300 may perform data communication not only with a server or a medical apparatus in a hospital, but also with a portable terminal of a medical doctor or patient.

The communication module 1300 is connected to the network 30 by wire or wirelessly to exchange data with a server 32, a medical apparatus 34, or a portable terminal 36. The communication module 1300 may include one or more components for communication with external devices. For example, the communication module 1300 may include a local area communication module 1310, a wired communication module 1320, and a mobile communication module 1330.

The local area communication module 1310 refers to a module for local area communication within a predetermined distance. Examples of local area communication techniques according to an embodiment may include, but are not limited to, wireless LAN, Wi-Fi, Bluetooth, ZigBee, Wi-Fi Direct (WFD), ultra wideband (UWB), infrared data association (IrDA), Bluetooth low energy (BLE), and near field communication (NFC).

The wired communication module 1320 refers to a module for communication using electric signals or optical signals. Examples of wired communication techniques according to an embodiment may include communication via a twisted pair cable, a coaxial cable, an optical fiber cable, and an Ethernet cable.

The mobile communication module 1330 transmits or receives wireless signals to or from at least one selected from a base station, an external terminal, and a server on a mobile communication network. The wireless signals may be voice call signals, video call signals, or various types of data for transmission and reception of text/multimedia messages.

The memory 1500 stores various data processed by the ultrasound imaging apparatus 1000. For example, the memory 1500 may store medical data related to diagnosis of an object 10, such as ultrasound data and an ultrasound image that are input or output, and may also store algorithms or programs which are to be executed in the ultrasound imaging apparatus 1002.

The memory 1500 may be any of various storage media, e.g., a flash memory, a hard disk drive, EEPROM, etc. Furthermore, the ultrasound imaging apparatus 1002 may utilize web storage or a cloud server that performs the storage function of the memory 1500 online.

The input unit 1600 refers to a means via which a user inputs data for controlling the ultrasound imaging apparatus 1002. Examples of the input unit 1600 may include For example, the input unit 1600 may include hardware components, such as a keyboard, a mouse, a touch pad, a touch screen, a trackball, and a jog switch, and software modules for operating the hardware components. However, embodiments are not limited thereto, and the input unit 1600 may further include any of various other input units including an electrocardiogram (ECG) measuring module, a respiration measuring module, a voice recognition sensor, a gesture recognition sensor, a fingerprint recognition sensor, an iris recognition sensor, a depth sensor, a distance sensor, etc.

Furthermore, the input unit 1600 may receive a user input for selecting an ROI included in the object 10. In this case, the user input may include a user's touch gesture. Examples of a user's touch gesture may include tap, touch & hold, double tap, drag, panning, flick, drag & drop, pinch, stretch, etc.

If the input unit 1600 is formed as a touch screen, the input unit 1600 may not only receive a user input but also perform the same function as the display 1400. In other words, the input unit 1600 may display and output an ultrasound image generated by the image processor 1200 and various pieces of information processed by the ultrasound imaging apparatus 1002 onto a screen via a graphical user interface (GUI). Furthermore, the input unit 1600 may display a model corresponding to the object 10 and an image showing an ROI generated by the image processor 1200 based on a user input.

The controller 1700 may control all operations of the ultrasound imaging apparatus 1000. In other words, the controller 1700 may control operations among the probe 20, the ultrasound transceiver 1100, the image processor 1200, the communication module 1300, the display 1400, the memory 1500, and the input 1600 shown in FIG. 1.

All or some of the probe 20, the ultrasound transceiver 1100, the image processor 1200, the communication module 1300, the display 1400, the memory 1500, the input 1600, and the controller 1700 may be implemented as software modules. Furthermore, at least one selected from the ultrasound transceiver 1100, the image processor 1200, and the communication module 1300 may be included in the controller 1600. However, embodiments of the present invention are not limited thereto.

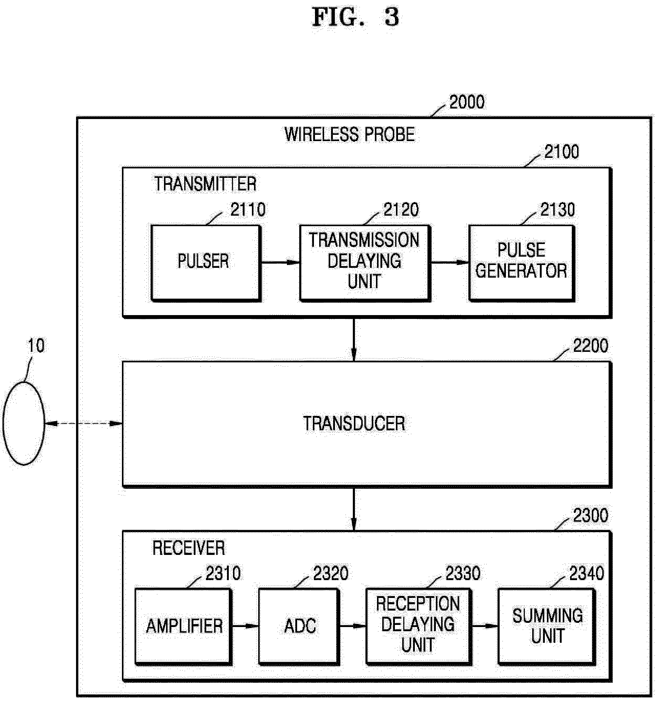

FIG. 3 is a block diagram of a configuration of a wireless probe 2000 according to an embodiment.

As described above with reference to FIG. 2, the wireless probe 2000 may include a plurality of transducers, and, according to embodiments, may include some or all of the components of the ultrasound transceiver 1100 shown in FIG. 2.

The wireless probe 2000 according to the embodiment shown in FIG. 2 includes a transmitter 2100, a transducer 2200, and a receiver 2300. Since descriptions thereof are given above with reference to FIG. 2, detailed descriptions thereof will be omitted here. In addition, according to embodiments, the wireless probe 2000 may selectively include a reception delaying unit 2330 and a summing unit 2340.

The wireless probe 2000 may transmit ultrasound signals to the object 10, receive echo signals from the object 10, generate ultrasound data, and wirelessly transmit the ultrasound data to the ultrasound imaging apparatus 1002 shown in FIG. 2.

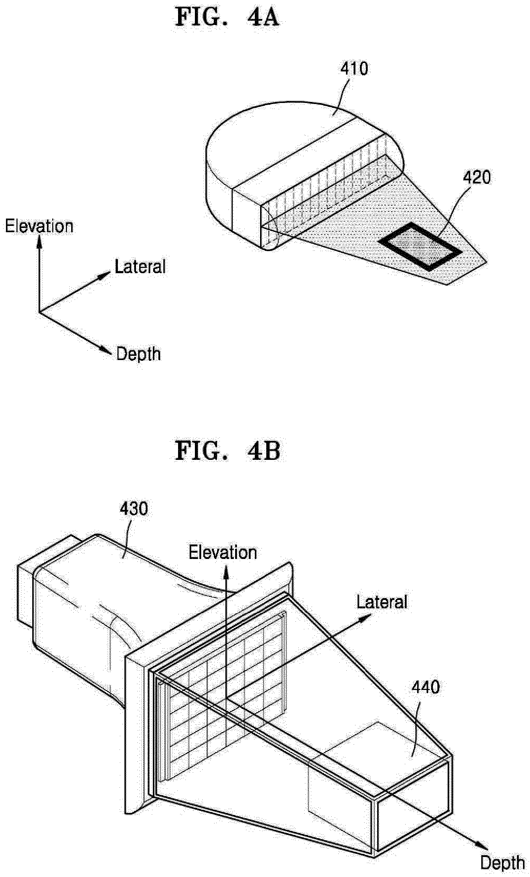

FIGS. 4A and 4B illustrate examples of one-dimensional (1D) and two-dimensional (2D) probes 410 and 430 according to embodiments.

The 1D probe 410 shown in FIG. 4A and the 2D probe 430 shown in FIG. 4B may respectively correspond to the probe 20 described with reference to FIG. 1A and the probe 20 or the wireless probe 2000 described with reference to FIG. 1B or 3.

Referring to FIG. 4A, the 1D probe 410 may be formed by a 1D array of a plurality of transducers. In this case, the transducers are elements constituting the 1D probe 410 and transmit ultrasound signals to an object 420 and receive echo signals reflected from the object 420. The plurality of transducers oscillate in response to the reflected echo signals, generate electrical pulses corresponding to the oscillations, and output the electrical pulses to the ultrasound transceiver 1100.

Furthermore, transducers in the 1D probe 410 may constitute an aperture or sub-array. In this case, the aperture is a set of some of the plurality of transducers in the probe 410. The number of transducers that constitute an aperture is not limited to a specific number, and one aperture may be composed of a single transducer.

Furthermore, referring to FIG. 4B, the 2D probe 430 may be formed by a 2D array of a plurality of transducers. The 2D probe 430 may transmit ultrasound signals to a 3D object 440 and receive echo signals reflected from the object 440. In this case, the 2D probe 430 may transmit ultrasound signals to the object 440 and receive echo signals in the same manner as described with reference to FIG. 4A.

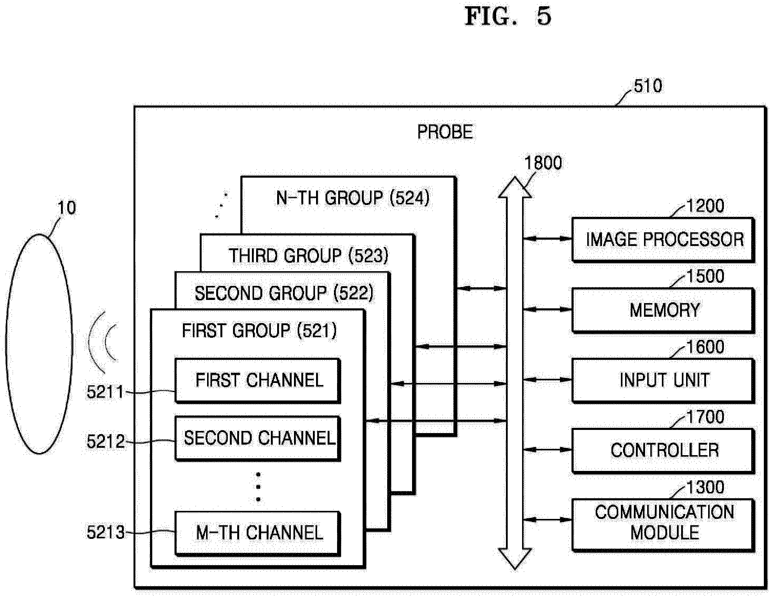

FIG. 5 illustrates a configuration of a probe 510 according to an embodiment.

The probe 510 of FIG. 5 may correspond to the 1D probe 410 described with reference to FIG. 4A or the 2D probe 430 described with reference to FIG. 4B.

The probe 510 transmits ultrasound signals to an object 10 (or to an ROI in the object 10) and receives echo signals reflected from the object 10 (or from the ROI in the object 10). Furthermore, the probe 510 may generate an ultrasound image by using ultrasound data corresponding to echo signals. In other words, the probe 510 may include the image processor 1200, the memory 1500, the input unit 1600, the controller 1700, and the communication module 1300 described with reference to FIG. 2, all of which may be connected to one another via the buses 1800.

The probe 510 includes a plurality of transducers, and the plurality of transducers oscillate in response to electric signals, generate acoustic energy, i.e., ultrasound waves, and receives echo signals reflected by the object 10 (or by the ROI in the object 10). In this case, each transducer is used to form a single channel, and may transmit an ultrasound signal and receive an echo signal according to an operation of an ultrasound transceiver included in each channel. In other words, each channel may include the ultrasound transceiver 1100 described with reference to FIG. 2.

Furthermore, a plurality of channels may be classified into a predetermined number of groups 521 through 524. For example, a first group 521 may include a plurality of channels 5211 through 5213, and the probe 510 may include the plurality of groups 521 through 524. Thus, signals generated by the plurality of channels 5211 through 5213 in the first group 521 may be summed before being transmitted to the image processor 1200.

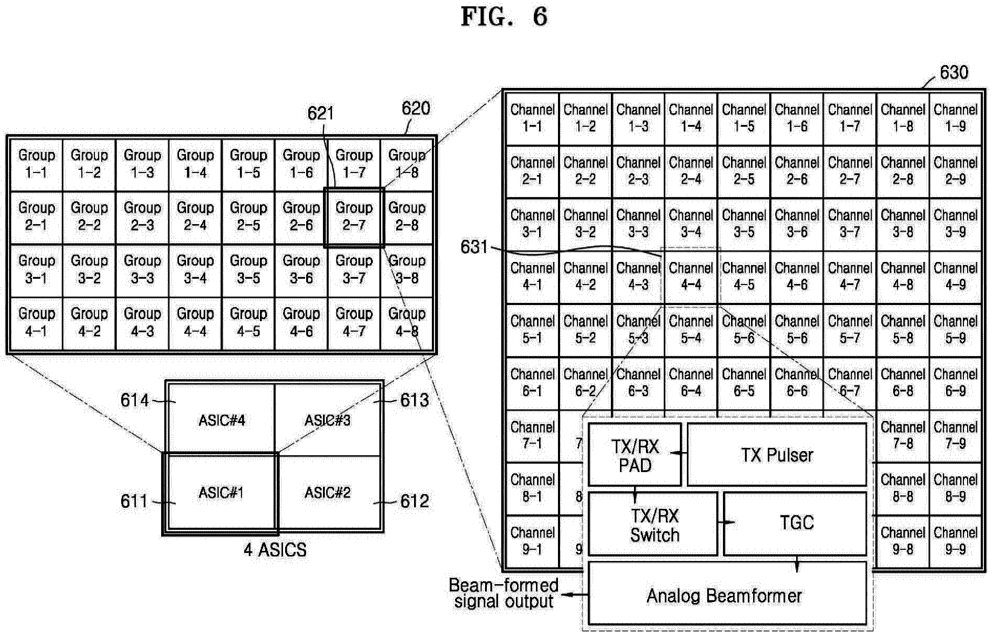

An example in which the probe 510 includes the plurality of groups 521 through 524, each group (e.g., 521) including a plurality of channels (e.g., 5211 through 5213), will now be described in detail with reference to FIG. 6.

FIG. 6 is a diagram for explaining an example of groups and channels in each group according to an embodiment.

Referring to FIG. 6, a probe may include a plurality of Application Specific Integrated Circuits (ASICs) 611 through 614, and one ASIC 611 may include a plurality of groups 620. While FIG. 6 shows that one ASIC 611 includes thirty-two (32) groups 620, the number of groups in the ASIC 611 is not limited to 32. The ASIC 611 outputs one output signal by beamforming signals output from the groups 620.

Furthermore, one group 621 may include a plurality of channels 630. Although FIG. 6 shows that the group 621 includes eighty-one (81) channels 630, the number of channels is not limited to 81. Thus, the ASIC 611 may include a plurality of channels.

One channel 631 corresponds to one transducer. In detail, the channel 631 generates transmission ultrasound waves transmitted by one transducer and processes echo signals received by the transducer to produce ultrasound data.

In other words, the channel 631 generates pulses for producing transmission ultrasound waves based on a PRF and delays the pulses by delay times necessary for determining transmission directionality. The channel 631 also applies a driving signal (or a driving pulse) to its corresponding transducer. For example, the channel 631 may include the transmitter 1110 or 2100 respectively described with reference to FIG. 2 or 3.

Furthermore, the channel 631 amplifies echo signals received from the transducer and performs analog-to-digital conversion (ADC) with respect to the amplified echo signals. The channel 631 may selectively perform time gain compensation (TGC) on digital echo signals. The channel 631 also delays the digital echo signals by delay times necessary for determining reception directionality to thereby generate ultrasound data. For example, the channel 631 may include the receiver 1120 or 2300 respectively described with reference to FIG. 2 or 3.

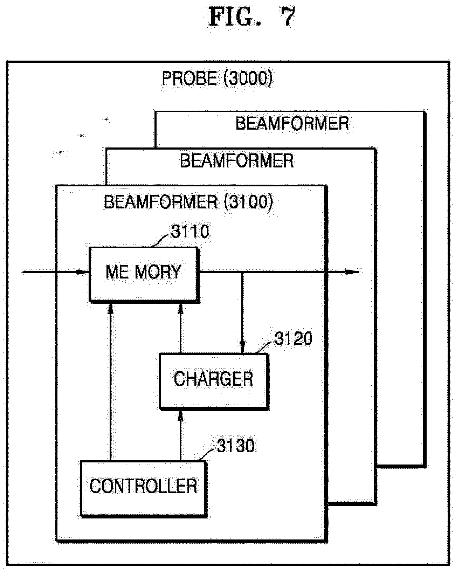

FIG. 7 illustrates a configuration of a beamformer 3100 according to an embodiment.

Referring to FIG. 7, the beamformer 3100 according to the present embodiment includes a memory 3110, a charger 3120, and a controller 3130. Although FIG. 7 shows that the beam former 3100 includes only components related to the present embodiment, it will be understood by those of ordinary skill in the art to which the present embodiment pertains that the beamformer 3100 may further include general-purpose components other than those shown in FIG. 7.

Furthermore, it will be appreciated by those of ordinary skill in the art that the memory 3110, the charger 3120, and the controller 3130 may operate independently of one another.

Furthermore, the beamformer 3100 may perform functions of the receiver 1120 or 2300 respectively described with reference to FIG. 2 or 3.

The beamformer 3100 may be embedded into a probe 3000. In other words, the beamformer 3100 may be connected to each of a plurality of channels in the probe 3000. For example, if the probe 3000 is a 1D probe, the beamformer 3100 may be connected to each of channels respectively corresponding to transducers in a 1D array. As another example, if the probe is a 2D probe, the beamformer 3100 may be connected to each of channels respectively corresponding to transducers in a 2D array.

The memory 3110 stores electric charges corresponding to echo signals reflected by the object (10 of FIG. 5). The memory 3110 also outputs stored electric charges. A transducer connected to the beamformer 3100 transmits ultrasound signals to the object 10 and receives echo signals acquired as the transmitted ultrasound signals are reflected by the object 10.The memory 3110 stores electric charges corresponding to amplitudes of the echo signals received by the transducer and outputs the stored electric charges in response to a control signal generated by the controller 3130.

The memory 3110 includes at least one capacitor and at least one switch. For example, a switch may be disposed between a transducer and a capacitor, and the controller 3130 may control a process of storing electric charges in a capacitor by controlling an operation of the switch. Furthermore, a switch may be disposed between a capacitor and an external device, and the controller 3130 may control a process of outputting electric charges from a capacitor by controlling an operation of the switch.

The charger 3120 generates an amount of electric charges corresponding to an amount of output electric charges. In other words, the charger 3120 generates the same amount of electric charges as those output from the memory 3110. The charger 3120 then transmits the generated electric charges to the memory 3110, and the memory 3110 stores the electric charges transmitted by the charger 3120.

For example, after electric charges are output from the memory 3110, the charger 3120 may transmit electric charges generated by the charger 3120 to the memory 3110. Thus, the same amount of electric charges as those output from the memory 3110 may be stored again in the memory 3110.

The controller 3130 controls operations of the memory 3110 and the charger 3120. For example, the controller 3130 may control a velocity at which electric charges are stored in the memory 3110 (hereinafter, referred to as a 'first velocity') and a velocity at which electric charges are output from the memory 3110 (hereinafter, referred to as a 'second velocity'). The controller 3130 may control the first velocity and/or the second velocity by controlling on/off states of at least one switch included in the memory 3110.

The ultrasound diagnosis system 1002 may have a high frame rate. In particular, when the ultrasound diagnosis system 1002 generates an image of the object 10 in motion, a frame rate is directly related to a quality of the image. The frame rate is inversely proportional to the number of transmissions of an ultrasound signal per frame. However, as the number of transmissions of an ultrasound signal increases, a signal having less distortion (i.e., a signal for generating a high quality image) may be obtained. Thus, it is necessary to provide a method of preventing distortion of a signal while reducing the number of transmissions of an ultrasound signal. To increase a frame rate, multi-beam reception focusing may be used. In the multi-beam reception focusing, a transducer transmits ultrasound signals once, and a plurality of scanlines may be generated simultaneously by using echo signals acquired as the transmitted ultrasound signals are reflected by the object 10.

In general, when beamforming is performed using a multi-beam reception focusing technique, a number of analog beamformers corresponding to the number of scanlines need to be provided in the probe 20 (2000) or the ultrasound imaging apparatus 100. Thus, the probe 20 or the ultrasound imaging apparatus 100 may become bulky. Furthermore, when beamforming is performed based on the multi-beam reception focusing technique, analog beamforming is performed for each sub-aperture, and a plurality of scanlines have to be generated during digital beamforming, which may rapidly degrade the quality of an ultrasound image.

When the ultrasound diagnosis system 1002 generates ultrasound data according to a multi-beam reception focusing method, the controller 3130 may control the second velocity based on the number of scanlines corresponding to ultrasound signals transmitted to the object 10. For example, the controller 3130 may control the second velocity based on Equation (1) below: ${F}_{read} = m * {F}_{write}$ where F_{read} is the frequency with which electric charges are input to the memory 3110 during a predetermined time interval Tₛ, F_{write} is the frequency with which electric charges are output from the memory 3110, and m is the number of scanlines. In other words, as shown in Equation (1), the controller 3130 may control the second velocity to be m times higher than the first velocity.

Furthermore, the controller 3130 may control the second velocity by taking into account delay values respectively calculated for a plurality of scanlines corresponding to ultrasound signals.

Furthermore, the controller 3130 may determine a time point at which electric charges generated by the charger 3120 are transmitted to the memory 3110 and control the charger 3120 to transmit the electric charges to the memory 3110 at the determined time point. For example, the controller 3130 may instruct the charger 3120 to transmit generated electric charges to the memory 3110 after electric charges are output from the memory 3110.

According to a multi-beam reception focusing method, electric charges stored in the memory 3110 may be used a plurality of times in order to generate a plurality of scanlines. For example, as seen in Equation (1), electric charges may be input to the memory 3110 once, and the same electric charges may be output from the memory 3110 m times. Since the charger 3120 included in the beamformer 3100 supplies electric charges to the memory 3110 a plurality of times, the beamformer 3100 may smoothly output electric charges used in generating a plurality of scanlines without interruption.

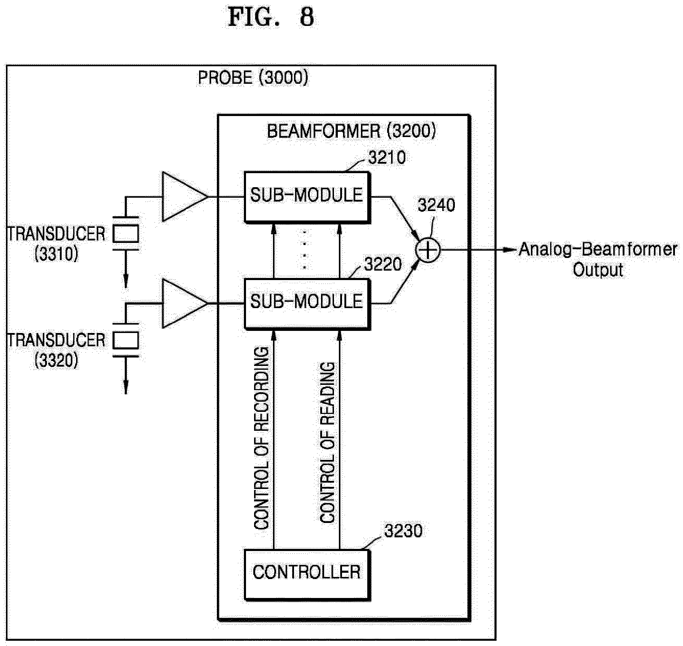

An example in which the beamformer 3100 is connected to a channel will now be described in detail with reference to FIG. 8.

FIG. 8 illustrates an example of a configuration in which a beamformer 3200 is connected to channels, according to an embodiment.

Referring to FIG. 8, the beamformer 3200 includes a plurality of sub-modules 3210 through 3220 respectively connected to transducers 3310 through 3320 in the probe 3000, and each of the sub-modules 3210 through 3220 is controlled by a controller 3230. Although FIG. 8 shows that the controller 3230 controls operations of the sub-modules 3210 through 3220, each of the sub-modules 3210 through 3220 may include an independently operating controller. Furthermore, as described with reference to FIG. 7, the beamformer 3200 may be embedded into the probe 3000.

Furthermore, each of the plurality of sub-modules 3210 through 3220 may include the memory 3110 and the charger 3120. Alternatively, each of the sub-modules 3120 through 3220 may include only the memory 3110, and one charger (e.g., the charger 3120) may be connected to all the sub-modules 3210 through 3220.

While FIG. 8 shows an example in which the beamformer 3200 is connected to one group including the transducers 3310 through 3320 in the probe 3000, embodiments are not limited thereto. The beamformer 3200 may be connected to a single transducer (e. g., the transducer 3310) or to all of a plurality of groups of transducers.

The transducer 3310 receives echo signals reflected by the object 10, and the sub-module 3210 stores electric charges corresponding to the echo signals. In this case, the controller 3230 may control a process of storing electric charges in the sub-module 3210 ('control of recording' in FIG. 8). Furthermore, the sub-module 3210 may output stored electric charges according to control by the controller 3230 ('control of reading' in FIG. 8). In addition, when electric charges are output from the sub-module 3210, the sub-module 3210 may generate an amount of electric charges corresponding to an amount of the output electric charges and store the generated electric charges.

In this way, electric charges may be output from the sub-modules 3210 through 3220 connected to the transducers 3310 through 3320 and be combined together (3240) and output from the beamformer 3200.

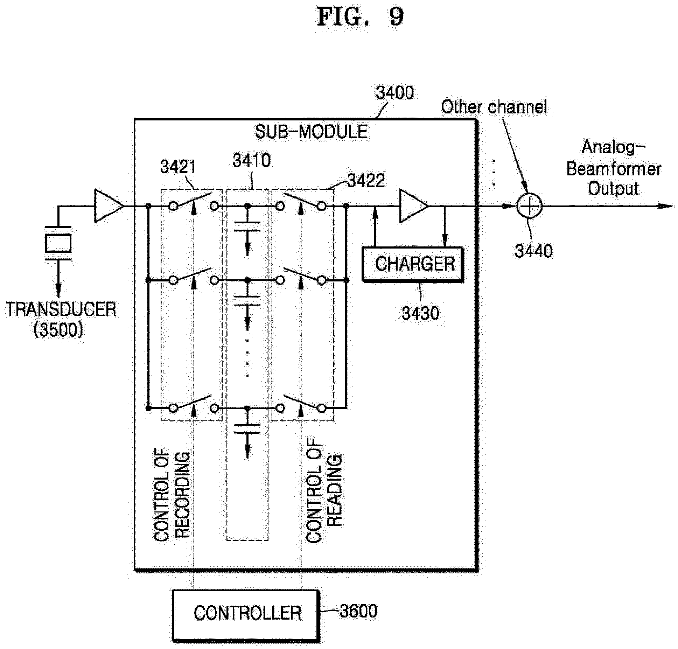

FIG. 9 illustrates a configuration of a sub-module 3400 according to an embodiment.

Referring to FIG. 9, the sub-module 3400 corresponds to the sub-module 3210 shown in FIG. 8. In other words, the sub-module 3400 may include a memory and a charger 3430. The memory may include a plurality of capacitors 3410 and a plurality of switches 3421 and 3422 connected to the capacitors 3410.

In addition, as shown in FIG. 9, although a controller 3600 is disposed outside the sub-module 3400, the controller 3600 may be located inside the sub-module 3400.

Electric charges are sequentially stored in the capacitors 3410 in response to echo signals received by a transducer 3500. In this case, a process of storing electric charges in the capacitor 3410 is performed as the switches 3421 are turned on/off according to control ('control of recording') by the controller 3600.

In addition, when ultrasound data is generated according to a multi-beam reception focusing method, electric charges stored in the capacitors 3410 are output selectively a plurality of times. In this case, a process of outputting electric charges in the capacitors 3410 is performed according to control ('control of reading') by the controller 3600. The controller 3600 may calculate delay values respectively for a plurality of scanlines and control on/off states of the switches 3422 so that electric charges stored in the capacitors 3410 may be output selectively a plurality of times based on the calculated delay values.

When electric charges are output from one of the capacitors 3410, the charger 3430 generates the same amount of electric charges as those output from the one of the capacitors 3410 and transmits the generated electric charges to the one of the capacitors 3410.

As described above with reference to FIG. 8, electric charges output from the sub-module 3400 may be combined (3400) together with electric charges output from other sub-modules and transmitted to another device.

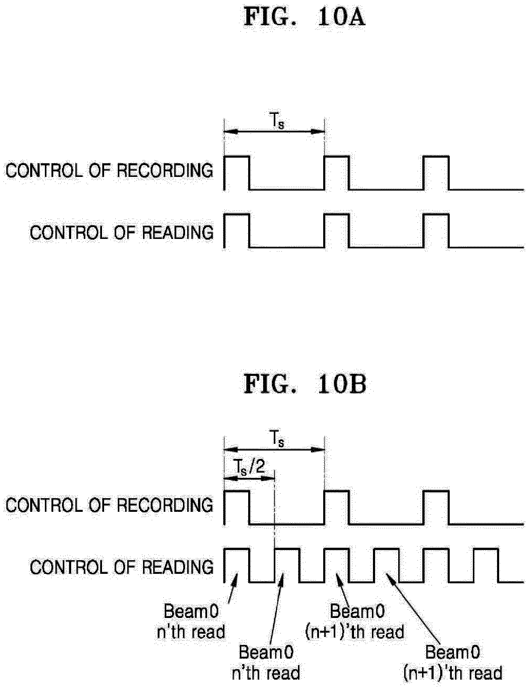

FIGS. 10A and 10B are diagrams for explaining an example in which a controller controls a velocity at which electric charges are input/output from a memory according to the number of scanlines.

FIG. 10A illustrates an example in which the controller controls, when a single scanline is generated, a process of storing electric charges in the memory (control of recording) and a process of outputting electric charges from the memory (control of reading).

Referring to FIG. 10A, during a predetermined time interval Tₛ, the number of times that electric charges are stored in the memory is equal to the number of times that electric charges are output from the memory. In other words, when a single scanline is generated, the controller controls the memory to output electric charges the same number of times as the number of times that electric charges are stored in the memory.

FIG. 10B shows an example in which the controller controls, when two scanlines are generated, a process of storing electric charges from the memory (control of recording) and a process of outputting electric charges from the memory (control of reading).

Referring to FIG. 10B, during a predetermined time interval Tₛ, the number of times that electric charges are stored in the memory is different from the number of times that electric charges are output from the memory. In other words, during the predetermined time interval Tₛ, when electric charges are stored once, the electric charges are output twice. In other words, the controller controls the memory to output the stored electric charges the same number of times as the number of scanlines. In this case, when electric charges are output from the memory, the charger transmits the same amount of electric charges as those output from the memory to the memory.

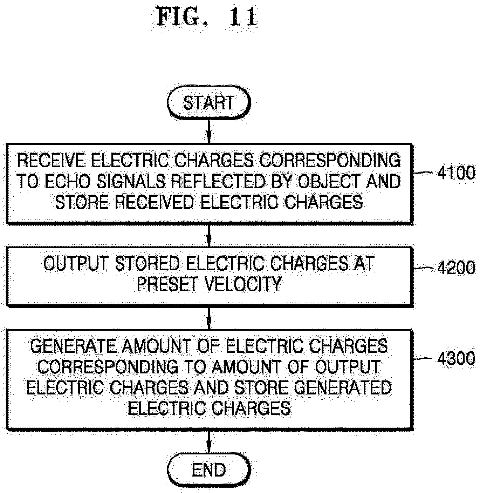

FIG. 11 is a flowchart of a method of performing beamforming according to an embodiment.

Referring to FIG. 11, the method includes operations sequentially performed by the beamformer 3100 or 3200 or the sub-module 3400 respectively shown in FIGS. 7, 8, and 9. Thus, although omitted hereinafter, the descriptions with respect to the beamformer 3100 or 3200 or the sub-module 3400 may apply to the method of FIG. 11.

A memory receives electric charges corresponding to echo signals reflected by an object and stores the received electric charges (operation 4100). For example, the memory may include at least one capacitor and at least one switch. Furthermore, the echo signals may be received by a 1D probe (i.e., a probe including a 1D transducer array) or a 2D probe (i.e., a probe including a 2D transducer array).

The memory outputs the stored electric charges at a preset velocity (operation 4200). For example, the velocity may be set based on the number of scanlines corresponding to ultrasound signals transmitted to the object. Furthermore, the velocity may be set to be m times higher than the first velocity if m represents the number of scanlines. Furthermore, the velocity may be set based on delay values respectively calculated for the scanlines.

A charger generates an amount of electric charges corresponding to an amount of the electric charges output from the memory (operation 4300). The charger then transmits the generated electric charges to the memory, and the memory stores the transmitted electric charges. For example, after the memory outputs electric charges, the charger may generate electric charges and transmit the generated electric charges to the memory.

According to the embodiments, by performing beamforming using a multi-beam reception focusing method, the number or size of necessary hardware components may be reduced and accordingly the probe or ultrasound imaging apparatus may become less bulky. Furthermore, it is possible to prevent degradation in quality of an ultrasound image.

## Claims

1. An ultrasound imaging probe performing beamforming using at least one analog beamformer (3100), comprising:
a memory (3110) comprising at least one capacitor (3410) to store electric charges corresponding to echo signals reflected by an object (10) and at least one switch (3421, 3422) to control the electric charges to be stored in the at least one capacitor (3410) and the stored electric charges to be outputted from the at least one capacitor (3410);
and
a controller (3130, 3600) configured to control at least one of a first velocity indicating a first frequency at which the electric charges are stored in the memory (3110) and a second velocity indicating a second frequency at which the electric charges are outputted from the memory (3110);
**characterized in that** the ultrasound imaging probe comprises:
a charger (3120, 3430) configured to generate electric charges corresponding to the outputted electric charges;
wherein the charger (3120, 3430) is further configured to generate the electric charges corresponding to the outputted electric charges, based on a capacitor from which the stored electric charges are outputted among the at least one capacitor (3410);
wherein the controller (3130, 3600) is further configured to control the second velocity, by controlling at least one operation of the at least one switch (3421, 3422), to be m times higher than the first velocity, where m represents a number of scanlines corresponding to ultrasound signals transmitted to the object (10).

2. The ultrasound imaging probe of claim 1, wherein the controller is further configured to control the second velocity based on delay values respectively calculated for the plurality of scanlines corresponding to ultrasound signals transmitted to the object.

3. The ultrasound imaging probe of claim 1, wherein the generated electric charges are inputted to the memory after the electric charges are outputted from the memory.

4. The ultrasound imaging probe of claim 1, further comprising a two-dimensional (2D) transducer array.

5. A method of performing beamforming in an ultrasound imaging probe comprising at least one analog beamformer, the method comprising:
receiving electric charges corresponding to echo signals reflected by an object and storing the received electric charges at first velocity indicating a first frequency at which the electric charges are stored in at least one capacitor in a memory (4100);
outputting the stored electric charges at a second velocity indicating a second frequency at which the electric charges are outputted from the at least one capacitor (4200); and
**characterized in that** the method comprises:
generating electric charges corresponding to the outputted electric charges and storing the generated electric charges (4300);
wherein the generating of the electric charges corresponding to the outputted electric charges comprises generating the electric charges corresponding to the outputted electric charges, based on a capacitor from which the stored electric charges are outputted among the at least one capacitor;
wherein the second velocity is set, by controlling at least one operation of at least one switch in the memory, to be m times higher than a velocity at which the electric charges are stored, where m represents a number of scanlines corresponding to ultrasound signals transmitted to the object.

6. The method of claim 5, wherein the second velocity is set based on delay values respectively calculated for the plurality of scanlines corresponding to ultrasound signals transmitted to the object.

7. The method of claim 5, wherein the echo signals are received by a probe comprising a two-dimensional (2D) transducer array.

## Patentansprüche

1. Ultraschallbildgebungssonde, die eine Strahlformung unter Verwendung zumindest eines analogen Strahlformers (3100) durchführt, umfassend:
einen Speicher (3110), der zumindest einen Kondensator (3410) zum Speichern elektrischer Ladungen, die von einem Objekt (10) reflektierten Echosignalen entsprechen, und zumindest einen Schalter (3421, 3422) zum Steuern der in dem zumindest einen Kondensator (3410) zu speichernden elektrischen Ladungen sowie der aus dem zumindest einen Kondensator (3410) auszugebenden elektrischen Ladungen umfasst;
und
eine Steuerung (3130, 3600), die so konfiguriert ist, dass sie zumindest eine der Folgenden steuert: eine erste Geschwindigkeit, die eine erste Frequenz angibt, mit der die elektrischen Ladungen in dem Speicher (3110) gespeichert werden, und eine zweite Geschwindigkeit, die eine zweite Frequenz angibt, mit der die elektrischen Ladungen aus dem Speicher (3110) ausgegeben werden;
**dadurch gekennzeichnet, dass** die Ultraschallbildgebungssonde Folgendes umfasst:
eine Ladeeinrichtung (3120, 3430), die so konfiguriert ist, dass sie elektrische Ladungen erzeugt, die den ausgegebenen elektrischen Ladungen entsprechen;
wobei die Ladeeinrichtung (3120, 3430) ferner so konfiguriert ist, dass sie die den ausgegebenen elektrischen Ladungen entsprechenden elektrischen Ladungen basierend auf einem Kondensator unter dem zumindest einen Kondensator (3410) erzeugt, aus dem die gespeicherten elektrischen Ladungen ausgegeben werden;
wobei die Steuerung (3130, 3600) ferner so konfiguriert ist, dass sie die zweite Geschwindigkeit durch Steuerung zumindest eines Schaltvorgangs des zumindest einen Schalters (3421, 3422) so steuert, dass sie m mal höher ist als die erste Geschwindigkeit, wobei m eine Anzahl von Abtastzeilen darstellt, die den an das Objekt (10) gesendeten Ultraschallsignalen entsprechen.

2. Ultraschallbildgebungssonde nach Anspruch 1, wobei die Steuerung ferner so konfiguriert ist, dass sie die zweite Geschwindigkeit basierend auf Verzögerungswerten steuert, die jeweils für die Vielzahl von Abtastzeilen berechnet werden, die den an das Objekt gesendeten Ultraschallsignalen entsprechen.

3. Ultraschallbildgebungssonde nach Anspruch 1, wobei die erzeugten elektrischen Ladungen in den Speicher eingeben werden, nachdem die elektrischen Ladungen aus dem Speicher ausgegeben wurden.

4. Ultraschallbildgebungssonde nach Anspruch 1, ferner umfassend eine zweidimensionale (2D) Wandleranordnung.

5. Verfahren zur Durchführung einer Strahlformung in einer Ultraschallbildgebungssonde, die mindestens einen analogen Strahlformer umfasst, wobei das Verfahren Folgendes umfasst:
Empfangen elektrischer Ladungen, die den von einem Objekt reflektierten Echosignalen entsprechen, und Speichern der empfangenen elektrischen Ladungen mit einer ersten Geschwindigkeit, die eine erste Frequenz angibt, mit der die elektrischen Ladungen in zumindest einem Kondensator in einem Speicher (4100) gespeichert werden;
Ausgeben der gespeicherten elektrischen Ladungen mit einer zweiten Geschwindigkeit, die eine zweite Frequenz angibt, mit der die elektrischen Ladungen aus dem zumindest einen Kondensator (4200) ausgegeben werden; und
**dadurch gekennzeichnet, dass** das Verfahren Folgendes umfasst:
Erzeugen elektrischer Ladungen, die den ausgegebenen elektrischen Ladungen entsprechen, und Speichern der erzeugten elektrischen Ladungen (4300);
wobei das Erzeugen der den ausgegebenen elektrischen Ladungen entsprechenden elektrischen Ladungen das Erzeugen der den ausgegebenen elektrischen Ladungen entsprechenden elektrischen Ladungen basierend auf einem Kondensator unter dem mindestens einen Kondensator umfasst, aus dem die gespeicherten elektrischen Ladungen ausgegeben werden;
wobei die zweite Geschwindigkeit durch Steuern mindestens eines Schaltvorgangs mindestens eines Schalters in dem Speicher so eingestellt wird, dass sie m mal höher ist als eine Geschwindigkeit, mit der die elektrischen Ladungen gespeichert werden, wobei m eine Anzahl von Abtastzeilen darstellt, die den an das Objekt gesendeten Ultraschallsignalen entsprechen.

6. Verfahren nach Anspruch 5, wobei die zweite Geschwindigkeit basierend auf Verzögerungswerten eingestellt wird, die jeweils für die Vielzahl von Abtastzeilen berechnet werden, die den an das Objekt gesendeten Ultraschallsignalen entsprechen.

7. Verfahren nach Anspruch 5, wobei die Echosignale an einer Sonde empfangen werden, die eine zweidimensionale (2D) Wandleranordnung umfasst.

## Revendications

1. Sonde échographique réalisant une formation de faisceau à l'aide d'au moins un formeur de faisceau analogique (3100), comprenant :
une mémoire (3110) comprenant au moins un condensateur (3410) destiné au stockage de charges électriques correspondant aux signaux d'écho réfléchis par un objet (10) et au moins un commutateur (3421, 3422) destiné à commander le stockage des charges électriques dans le ou les condensateurs (3410) et la sortie des charges électriques stockées du ou des condensateurs (3410) ;
et
un organe de commande (3130, 3600) conçu pour commander une première vitesse indiquant une première fréquence à laquelle les charges électriques sont stockées dans la mémoire (3110) et/ou une deuxième vitesse indiquant une deuxième fréquence à laquelle les charges électriques sortent de la mémoire (3110) ;
**caractérisée en ce que** la sonde échographique comprend :
un chargeur (3120, 3430) conçu pour générer des charges électriques correspondant aux charges électriques de sortie ;
ledit chargeur (3120, 3430) étant en outre conçu pour générer les charges électriques correspondant aux charges électriques de sortie compte tenu d'un condensateur, parmi ledit au moins un condensateur (3410), duquel sortent les charges électriques stockées ;
ledit organe de commande (3130, 3600) étant en outre conçu pour commander la deuxième vitesse, en commandant au moins une opération réalisée par le ou les commutateurs (3421, 3422), de façon qu'elle soit m fois supérieure à la première vitesse, m représentant un nombre de lignes de balayage correspondant aux signaux ultrasonores émis vers l'objet (10).

2. Sonde échographique selon la revendication 1, dans laquelle l'organe de commande est en outre conçu pour commander la deuxième vitesse en fonction de valeurs de retard respectivement calculées pour la pluralité de lignes de balayage correspondant aux signaux ultrasonores émis vers l'objet.

3. Sonde échographique selon la revendication 1, dans laquelle les charges électriques générées sont mises en mémoire après que les charges électriques sont sorties de la mémoire.

4. Sonde échographique selon la revendication 1, comprenant en outre une matrice bidimensionnelle (2D) de transducteurs.

5. Procédé de réalisation d'une formation de faisceau dans une sonde échographique comprenant au moins un formeur de faisceau analogique, le procédé comprenant :
la réception de charges électriques correspondant à des signaux d'écho réfléchis par un objet et le stockage des charges électriques reçues à une première vitesse indiquant une première fréquence à laquelle les charges électriques sont stockées dans au moins un condensateur d'une mémoire (4100),
la sortie des charges électriques stockées, à une deuxième vitesse indiquant une deuxième fréquence à laquelle les charges électriques sortent de l'au moins un condensateur (4200) ; et
**caractérisé en ce que** le procédé comprend :
la génération de charges électriques correspondant aux charges électriques de sortie et le stockage des charges électriques générées (4300) ;
ladite génération des charges électriques correspondant aux charges électriques de sortie comprenant la génération des charges électriques correspondant aux charges électriques de sortie compte tenu d'un condensateur, parmi ledit au moins un condensateur, duquel sortent les charges électriques stockées ;
ladite deuxième vitesse étant définie, par commande d'au moins une opération de l'au moins un commutateur de la mémoire, pour être m fois supérieure à la vitesse à laquelle les charges électriques sont stockées, m représentant un nombre de lignes de balayage correspondant aux signaux ultrasonores émis vers l'objet.

6. Procédé selon la revendication 5, dans lequel la deuxième vitesse est définie en fonction de valeurs de retard respectivement calculées pour la pluralité de lignes de balayage correspondant aux signaux ultrasonores émis vers l'objet.

7. Procédé selon la revendication 5, dans lequel les signaux d'écho sont reçus par une sonde comprenant une matrice bidimensionnelle (2D) de transducteurs.
